(19) Europäisches Patentamt
European Patent Office
Office européen des brevets

(11) **EP 3 453 385 A1**

(12) **EUROPÄISCHE PATENTANMELDUNG**

(43) Veröffentlichungstag:
**13.03.2019 Patentblatt 2019/11**

(51) Int Cl.:
***A61K 9/50*** *(2006.01)* ***A61K 9/51*** *(2006.01)*

(21) Anmeldenummer: **17189860.4**

(22) Anmeldetag: **07.09.2017**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(71) Anmelder: **Justus-Liebig-Universität Gießen**
**35390 Gießen (DE)**

(72) Erfinder:
• **Gessler, Tobias**
**35435 Wettenberg (DE)**

• **Dalla-Bona, Alexandra**
**35452 Heuchelheim (DE)**
• **Rajkumar, Savai**
**61231 Bad Nauheim (DE)**
• **Savai Pullamsetti, Soni**
**61231 Bad Nauheim (DE)**
• **Schermuly, Ralph**
**35794 Mengerskirchen (DE)**
• **Seeger, Werner**
**35444 Biebertal (DE)**

(74) Vertreter: **Stumpf, Peter**
**c/o TransMIT GmbH**
**Kerkrader Strasse 3**
**35394 Gießen (DE)**

(54) **ERFINDUNG BETREFFEND NANOPARTIKEL ENTHALTEND TAXANE ZUR INHALATIVEN APPLIKATION**

(57) Die Erfindung betrifft Nanopartikel zur inhalativen Applikation des Wirkstoffes, ausgewählt aus der Liste umfassend Paclitaxel, Docetaxel sowie SB-T-1214, umfassend PLGA, PVA, TPGS, PLA und zur Therapie der pulmonalen Hypertension. Die Partikel zeichnen sich durch einen hohen Beladungsgrad sowie große Stabilität im Vernebelungsprozess mit einem Verneblungsgerät aus.

Fig. 5A

**Beschreibung**

Die vorliegende Erfindung betrifft die Gebiete Innere Medizin, Pharmakologie und Nanotechnologie. Die pulmonale Hypertonie (PH) ist eine schwere, lebensbedrohliche Erkrankung des pulmonalen Gefäßsystems von Menschen, die die körperliche Belastbarkeit stark einschränkt. Der Anstieg des pulmonalen arteriellen Drucks und des vaskulären Widerstands mit nachfolgender Rechtsherz-Dysfunktion resultiert in einer stark reduzierten Lebenserwartung im Falle der Unterform der pulmonal arteriellen Hypertonie (PAH) beträgt die durchschnittliche Überlebenszeit ohne Behandlung nur 2,8 Jahre nach Diagnosestellung.

In den letzten Jahren wurden verschiedene gefäßaktive Wirkstoffe zur Behandlung dieser Erkrankung zugelassen, die jedoch zu keiner Heilung oder anhaltenden Prognoseverbesserung führen. Die bisher verfügbaren Medikamente adressieren die grundlegenden Stoffwechselwege der pulmonalen Gefäßregulationen: den Prostazyklin-, Stickstoffmonoxid (NO)- und Endothelin-Pathway. Aus der Gruppe der Prostazykline sind Epoprostenol (intravenös) sowie die stabilen Prostazyklin-Analoga Iloprost (intravenös und inhalativ), Treprostinil (intravenös, subkutan, inhalativ) und Beraprost (oral) zugelassen, darüber hinaus Selexipag als selektiver Agonist des Prostazyklin-IP-Rezeptors für die orale Applikation. Medikamente aus dem Stoffwechselweg von NO sind die oralen Phosphodiesterase-5-Inhibitoren Sildenafil und Tadalafil. Mit Riociguat steht ein erster Wirkstoff aus der Klasse der sGC-Stimulatoren bzw. sGC-Aktivatoren für die Therapie der PH zur Verfügung. Bosentan, Ambrisentan und Macitentan sind zugelassene orale Arzneimittel aus der Gruppe der Endothelinrezeptor-Antagonisten.

Neue Forschungsergebnisse zeigen, dass die pulmonale Hypertonie mit proliferativen, anti-apoptotischen und damit tumorähnlichen Veränderungen von pulmonalen Widerstandsgefäßen einhergeht. Die Forkhead box O (FoxO)-Transkriptionsfaktoren nehmen dabei eine Schlüsselrolle in der Kontrolle der zellulären Proliferation ein, in erkrankten Gefäßen sind diese herunterreguliert bzw. inaktiviert.

Es wurde vorgeschlagen, dass die pharmakologische Rekonstitution der FoxO-Aktivität durch den bekannten Stoff Paclitaxel zu einem Re-Remodeling der pathologisch veränderten pulmonalen Widerstandsgefäße und damit zu einer signifikanten Verbesserung der pulmonalen Hypertonie führt.

Paclitaxel ist ein bekanntes Chemotherapeutikum aus der Gruppe der Taxane und steht als Abraxane® bzw. Taxol® zur intravenösen Behandlung verschiedener Tumorerkrankungen zur Verfügung. Allerdings limitieren die jeweils beigefügten Hilfsstoffe des in Wasser nur schwer löslichen Paclitaxel dessen pulmonale Applikation. Im Falle von Taxol® schädigt der beigefügte Lösungsvermittler Macrogolglycerolricinoleat (Cremophor) die für den Gasaustausch essentielle Funktion des pulmonalen Surfactantsystems. Im Falle von Abraxane® ist Paclitaxel an Albumin-Nanopartikel einer mittleren Größe von ungefähr 130 Nanometer gebunden. In Heilversuchen mit inhalativer Applikation von vernebeltem Abraxane® bei Patienten mit schwerer PH zeigte sich eine initial sehr gute Verträglichkeit des Medikaments. Bei repetitiver Gabe des Medikaments entwickelte sich jedoch ein Hypersensitivitätssyndrom mit allergischen Reaktionen auf das in Abraxane® verwendete Humanalbumin, sodass eine therapeutische Anwendung des Abraxane® zur Aerosoltherapie der PH nicht möglich ist, wie sie zum Beispiel in US-Patent 2014/0271871 A1 vorgeschlagen wurde.

Die inhalative Applikation ist die Verabreichung von Wirkstoffen in Form eines Aerosols über Inhalation in die Lunge, auch pulmonale Applikation genannt. Sie ist eine geeignete Möglichkeit, um dem Patienten eine leicht anwendbare Verabreichung zu ermöglichen. Unerwünschte systemische Nebenwirkungen, die mit der intravenösen oder oralen Applikation verbunden sind, werden dem Patienten erspart. Weiterhin kann die eingesetzte Wirkstoffmenge deutlich reduziert werden, da bei der inhalativen Applikation der Wirkstoff direkt an die Lunge gebracht wird und nicht erst eine Verteilung und Verdünnung durch den ganzen Körper stattfindet. Besonders vorteilhaft ist die inhalative Applikation bei der Verabreichung von Wirkstoffen zur Therapie von Lungenerkrankungen.

Das Europäische Arzneibuch unterscheidet folgende Zubereitungen, die zur inhalativen Applikation geeignet sind:

- Zubereitungen, die in Dampf überführt werden
- Flüssige Zubereitungen zur Verneblung
- Flüssige Zubereitungen in Druckgas-Dosierinhalatoren
- Pulver zur Inhalation

Das Europäische Arzneibuch nennt als Geräte, die die Zubereitungen zur Inhalation verabreichen können:

- Vernebler
- Inhalatoren
- Druckgas-Dosierinhalator
- Normaldruck-Dosierinhalator
- Pulver-Inhalator

Es besteht der Bedarf an einer Möglichkeit, Paclitaxel und andere Taxane wie Docetaxel bzw. Second Generation Taxane wie SB-T-1214 so zu formulieren und eine Zubereitung zur Verfügung zu stellen, um diese der inhalativen Applikation zugänglich zu machen, z.B. den Wirkstoff als Aerosol inhalativ applizieren zu können. Damit könnten Patienten mit pulmonaler Hypertonie (PH) erstmalig Wirkstoffe und Therapeutika zur Verfügung gestellt werden, die aufgrund ihrer Antitumoreigenschaften eine bessere Wirksamkeit versprechen. Die direkte inhalative Applikation des Wirkstoffs in die Lunge erleichtert die gezielte und nebenwirkungsarme Behandlung der pulmonalen Hypertonie.

Zahlreiche Materialien wie z. B. biokompatible Nanopartikel sind als geeignete Wirkstoffträgersysteme für das sogenannte Drug-Delivery auch für Taxane seit langem bekannt. Im Falle von Paclitaxel sind nanopartikuläre Formulierungen für die intravenöse Tumortherapie beschrieben (z. B. Bernabeu et al.; Paclitaxel: What has been done and the challenges remain ahead, Int J Pharm. 2017 526(1-2):474-495). Diese bekannten Nanopartikel eignen sich zwar für die intravenöse Anwendung. Sie sind jedoch nicht stabil gegenüber Verneblung und daher nicht zur inhalativen Applikation oder zur Verwendung in einem Vernebler geeignet.

Für die pulmonale Applikation von anderen Wirkstoffen wie Treprostinil, Iloprost sind zwar nanopartikuläre Trägersysteme bekannt, allerdings sind diese nicht für die Verwendung von Taxanen wie Docetaxel bzw. Second Generation Taxane wie SB-T-1214 und insbesondere Paclitaxel geeignet, weil sie den Wirkstoff Taxane nicht aufnehmen können, da er nicht wasserlöslich ist. Diese Trägersysteme sind nicht vernebelbar und auch nicht stabil gegenüber dem Prozess der Aerosolerzeugung, der eine hohe mechanische Belastung durch Druckbeaufschlagung, Ultraschall- und/oder thermische Behandlung aufweist.

Derzeit existieren keine vernebelbaren Taxanformulierungen auf der Basis von biokompatiblen Nanopartikeln, die zur inhalativen Applikation geeignet sind.

Wünschenswert wäre der direkte Transport von in Nanopartikeln verkapselten Taxanen wie Docetaxel bzw. Second Generation Taxane wie SB-T-1214 insbesondere Paclitaxel über eine inhalative Applikation in die Lunge, sodass eine kontrollierte Wirkstoff-Freigabe am Wirkort Lunge erreicht wird, um die Behandlung der pulmonalen Hypertonie mit Taxanen wie Docetaxel bzw. Second Generation Taxane wie SB-T-1214 insbesondere Paclitaxel zu ermöglichen.

## Aufgabe

Aufgabe der vorliegenden Erfindung ist es biokompatible Nanopartikel für die inhalative Applikation von Wirkstoff wie Paclitaxel und anderen Taxanen wie Docetaxel bzw. Second Generation Taxane wie SB-T-1214 bereitzustellen, die zur inhalativen Applikation und zur Behandlung der pulmonalen Hypertonie geeignet sind. Die Partikel sollen leicht herstellbar sein, einen hohen Beladungsgrad an Wirkstoff aufweisen, lagerungsbeständig und stabil gegenüber den Prozessen der Aerosolerzeugung sein, wie beispielsweise Druckbeaufschlagung, Ultraschall- und/oder thermische Behandlung.

## Lösung der Aufgabe

Diese Aufgabe wird erfindungsgemäß gelöst durch die Nanopartikel gemäß Anspruch 1. Besondere Ausführungsformen befinden sich in den untergeordneten Ansprüchen.

Überraschenderweise ist es gelungen, biokompatible Nanopartikel bereitzustellen, die den Wirkstoff Paclitaxel oder auch andere Taxane wie Docetaxel bzw. Second Generation Taxane wie SB-T-1214 enthalten, die als Aerosol vernebelbar sind und damit zur inhalativen Applikation sehr gut geeignet sind.

Vorteilhafterweise weisen die Partikel einen hohen Beladungsgrad an Wirkstoff auf und sind leicht herstellbar. Sie sind stabil gegenüber dem Prozess der Aerosolerzeugung, also Druckbeaufschlagung, Ultraschall- und/oder thermischer Behandlung. Die Lagerungsbeständigkeit der erfindungsgemäßen Partikel zeigt sich darin, dass die physikochemischen Eigenschaften wie Größe, Zetapotential und Beladung nach 11 Monaten nicht wesentlich verändert haben. Es kann eine Lagerungsbeständigkeit von mindestens 11 Monaten gezeigt werden.

Die erfindungsgemäßen Nanopartikel werden aus den bioabbaubaren Polymeren PLGA (Poly-(lactid-co-glycolid)), PLA (Polylactide), PLA-PEO-PLA (Poly(lactide -b-ethylene oxide -b-lactide), zusammen mit einem alpha-Tocopherolderivat TPGS (D-alpha-Tocopheryl Polyethylenglycol 1000 Succinat) bzw. TPGS-750-M (und DL-$\alpha$-Tocopherol methoxypolyethylene glycol succinate und PVA (Poly-(vinylalkohol) in verschiedenen Kompositionen mittels einer modifizierten Lösungsmittel-Verdampfungsmethode hergestellt. Es entstehen PVA-beschichtete Wirkstoff-haltige biokompatible Nanopartikel. In einer Ausführungsform liegen Paclitaxel-haltige Nanopartikel vor. In weiteren Ausführungsformen Docetaxel enthaltende Nanopartikel oder Second Generation Taxan (SB-T-1214) enthaltende Nanopartikel.

Insbesondere umfassen die Nanopartikel mindestens drei Polymere, unabhängig voneinander ausgewählt aus der Liste umfassend PLGA, PLA, TPGS, TPGS-750M, PVA, LAEOLA/PLA-PEO-PLA sowie mindestens einen Wirkstoff, ausgewählt aus der Liste umfassend Paclitaxel, Docetaxel sowie SB-T-1214.

Die Partikel weisen einen Kern-Schale-Aufbau auf, wobei der Kern den mindestens einen Wirkstoff und mindestens zwei Polymere enthält und diese Polymere unabhängig voneinander ausgewählt sind aus der Liste umfassend RG502H, RG504, PLA, LAEOLA/PLA-PEO-PLA, TPGS sowie TPGS-750M. Die Schale enthält PVA und/oder TPGS.

Diese PVA-beschichteten Wirkstoff-haltigen Nanopartikel weisen eine hohe Beladungseffizienz von Wirkstoff bezogen auf das Partikelgesamtgewicht zwischen 1% und 80%, bevorzugt zwischen 3% und 70%, besonders bevorzugt zwischen 5% und 50%, ganz besonders bevorzugt zwischen 10% und 40% auf. Die Größe liegt bei 200-3000 nm.

Die Nanopartikel liegen nach der Herstellung als Nanopartikelsuspension vor. Diese Nanopartikelsuspension wird mittels eines konventionellen Aerosolgenerators z.B. eines piezoelektrischen Aerosolgenerators als Aerosol vernebelt, wobei die physikochemischen Eigenschaften der Nanopartikel erhalten bleiben. Die Partikel zeigen eine kompositionsabhängige Freisetzung von Wirkstoff, wie dies exemplarisch für Paclitaxel in *in vitro*-Experimenten gezeigt wird. Darüber hinaus zeigt sich in Zellkulturen der pulmonalen Hypertonie (menschliche IPAH-PASMC-Zellen) ein therapeutischer Effekt. Damit erweisen sich die erfindungsmäßen Nanopartikel und die daraus hergestellte Nanopartikelsuspension als geeignet zur Verneblung und damit zur inhalativen Applikation von Paclitaxel, Docetaxel Second Generation Taxan (SB-T-1214). Sie stellen ein geeignetes Therapeutikum zur Behandlung der pulmonalen Hypertonie dar.

Ein weiterer Aspekt der Erfindung ist ein Verfahren zur Herstellung der erfindungsgemäßen Nanopartikel.

Es handelt sich bei den erfindungsgemäßen Nanopartikeln um Hybrid-Matrix-Nanopartikel, die aus einer Kombination von biokompatiblen Polymeren, Wirkstoff (ausgewählt aus der Gruppe Paclitaxel, Docetaxel, Second Generation Taxane wie SB-T-1214) und einem Tocopherolderivat in ihrer Matrix und einem schützenden PVA-Mantel (Coat) bestehen. Der Wirkstoff (ausgewählt aus der Gruppe Paclitaxel, Docetaxel, Second Generation Taxane wie SB-T-1214) ist ein äußerst komplexer Wirkstoff, da er in wässrigen Medien nur schwer löslich ist und sich rasch zersetzt. Die Herstellung aus wässrigem Medium ist daher nicht möglich. Bezogen auf das Lösungsmittel-Verdampfungsverfahren gibt es zusätzlich die Einschränkung, dass die Verwendung von polaren organischen Lösungsmitteln (Ethanol, Aceton) zum Aushärten des Wirkstoffes in seine kristalline Form (nadelförmige Kristalle) führt: Da Ethanol und Aceton wasserlöslich sind, lösen sich diese beiden Lösungsmittel sofort in der entstehenden wässrigen Mischung, wodurch der Wirkstoff ausfällt, und zwar in sehr undefinierter kristalliner Form, deren biologische Verfügbarkeit nicht einstellbar ist. Dies tritt zwar langsamer, möglicherweise sogar nur unvollständig auch bei polaren organischen Lösungsmitteln auf, die nur teilweise, bzw. wenig in Wasser löslich sind, es führt aber dennoch zu undefinierten pharmakologisch nicht verwendbaren Partikeln. Es ist also wichtig, non-polare organische Lösungsmittel wie z.B. Dichlormethan zu verwenden, um den Wirkstoff in seiner amorphen Struktur zu erhalten. PVA als Coat und das Tocopherolderivat reagieren in diesem Fall synergistisch, um das Auskristallisieren des Wirkstoffes zu verhindern. Das PVA-Coating schützt zusätzlich die Partikel gegen den Einfluss der kinetischen Energie des Vernebelns und ermöglicht eine lange Lagerungsstabilität als wässrige Suspension, was von Vorteil im Hinblick auf eine Verneblung in vibrating mesh nebulizern ist (nur Flüssigkeiten sind vernebelbar). Ein Poloxamer-Coating eignet sich aufgrund der hohen Wasserlöslichkeit und der Inkompatibilität mit dem Lungengewebe nicht.

Ein weiterer Aspekt ist die Notwendigkeit eines verlässlichen Puffers, um einen neutralen pH von 7,4 einzustellen. Polymere auf Milchsäure- bzw. Glycolsäurebasis neigen bei leicht sauren bis sauren Bedingungen zur autokatalytischen Spaltung in die enthaltenen Monomere. Demnach muss bei der Herstellung ein konstant neutraler pH herrschen, um die Konformität der Polymere zu wahren. Überraschenderweise wurde als geeigneter Puffer HEPES gefunden. HEPES ist eine dem Fachmann bekannte Kurzbezeichnung und steht für 2-(4-(2-Hydroxyethyl)-1-piperazinyl)-ethansulfonsäure. HEPES zeichnet sich durch eine gute Verträglichkeit für den Menschen aus, da er nicht durch Zellmembranen diffundiert und u.a. deshalb keine Gefahrstoffkennzeichnung (GHS) trägt. Weiterhin geht HEPES als Puffersubstanz kaum Wechselwirkungen mit Polymeren ein. Er zeigt eine hohe Löslichkeit und ist sehr kostengünstig herzustellen.

Die verwendeten Polymere PLA und PLGA sind einzeln eingesetzt ungeeignet, um den Wirkstoff in ausreichender Art zu verkapseln, da sie keine geeigneten Partikeleigenschaften erzeugt werden können. Erst die Kombination von PLA und PLGA führt zu sehr guten Partikeleigenschaften und zu einer sehr guten Beladung.

Darüber hinaus kann durch das Mischungsverhältnis das gewünschte Freisetzungsprofil erreicht werde. Trägerstoff und Stabilisator in der Matrix sind hierbei PLGA (in unterschiedlichen Monomerverhältnissen und funktionellen Endgruppen) und TPGS bzw TPGS 750-M. Durch Einbringen hydrophiler Komponenten wie PLA oder Triblock-Copolymere wie PLA-PEO-PLA können porenartige Strukturen geschaffen werden, durch die der Wirkstoff gezielt freigesetzt werden kann (sustained release).

Final ist alternativ eine Aufreinigung und anschließende Filtration der Nanopartikelsuspension möglich, um vernebelbare Partikelsuspensionen zu erhalten. Zum einen kann so das Entfernen von organischen Lösungsmittelrückständen sichergestellt werden, zum anderen werden PVA-Reste und andere Polymerrückstände entfernt, die evtl. die Verneblung durch Verstopfen der Verneblerporen beeinträchtigen würden.

**Allgemeine Vorschrift zur Herstellung erfindungsgemäßer Nanopartikel**

Die Nanopartikel werden in einem einfach durchführbaren Verfahren hergestellt, das folgende Schritt umfasst:

a) Herstellen einer wässrigen Phase umfassend 0,01 % bis 1%, bevorzugt 0,05%, eines Polymers, ausgewählt aus der Liste umfassend PVA sowie TPGS und/oder der Puffersubstanz HEPES, wobei das Polymer und/oder die Puffersubstanz HEPES unter Rühren gelöst wird und der pH-Wert auf einen Wert zwischen 7 und 8, bevorzugt auf etwa 7,4 eingestellt und die wässrige Phase auf 19°C bis 25°C, bevorzugt auf 22°C abgekühlt wird Alternativ wird die wässrige Phase nach der Herstellung zusätzlich filtriert.

b) Herstellen einer organischen Phase umfassend

- mindestens zwei Polymeren ausgewählt aus der Liste umfassend RG502H, RG504, LAEOLA, PLA, TPGS sowie TPGS-750M wobei die Konzentration der mindestens zwei Polymere 10 mg/ml bis 30 mg/ml, bevorzugt etwa 15 mg/ml beträgt
- mindestens einen Wirkstoff ausgewählt aus der Liste umfassend Paclitaxel, Docetaxel sowie SB-T-1214 wobei die Konzentration des mindestens einen Wirkstoffs 5 mg/ml bis 15 mg/ml, bevorzugt etwa 10 mg/ml beträgt

wobei die mindestens zwei Polymere und der mindestens eine Wirkstoff in einem unpolaren aprotischen organischen Lösungsmittel, ausgewählt aus der Liste umfassend Methylenchlorid sowie Ethylacetat gelöst werden.

Alternativ wird die wässrige Phase nach der Herstellung zusätzlich filtriert.

c) Mischen von 5 Teilen der wässrigen Phase aus Schritt a) mit einem Teil der organischen Phase aus Schritt b) sodass eine Emulsion entsteht, die bei 0°C bis 30°C, bevorzugt bei 0°C bis 4°C für einen Zeitraum von 1 bis 5 Minuten, bevorzugt etwa 3 Minuten mit einem Hochleistungs-Dispergiergerät bei 10.000 Umdrehungen/Min. bis 30.000 Umdrehungen/Min., bevorzugt bei etwa 21.000 Umdrehungen/Min fein emulgiert wird, so dass die Teilchengröße der Emulsion weiter verringert wird.

Alternativ entsteht die Emulsion bei einer Temperatur von 19-22°C, unter ständigem Rühren bei einer Rührgeschwindigkeit zwischen 300 Umdrehungen/Min. und 700 Umdrehungen/Min., bevorzugt etwa 500 Umdrehungen/Min.

d) Homogenisieren der Emulsion aus Schritt c) mit einem Ultraschall-Homogenisator bei 0°C bis 30°C, bevorzugt bei 0°C bis 4°C,

Alternativ erfolgt das Homogenisieren zunächst 0,5 bis 2 Minuten, bevorzugt 1 Minute, mit einer Leistung von etwa 25 Watt und anschließend 0,5 bis 2 Minuten, bevorzugt 1 Minute, mit einer Leistung von etwa 30 Watt.

In einer weiteren Alternative erfolgt das Homogenisieren 2 bis 5 Minuten, bevorzugt 3 Minuten, mit einer Leistung von 20 bis 40 Watt, bevorzugt mit einer Leistung von 25 bis 30 Watt oder mit einer Amplitude von 20% bis 90%, bevorzugt mit einer Amplitude von 30% bis 75%, besonders bevorzugt mit einer Amplitude von 60% bis 75% erfolgt.

Alternativ erfolgt die Ultraschall-Homogenisierung mit oder ohne Pulsieren, wobei das Verhältnis "Dauer der Pause" und "Dauer der Schallwirkung" beim Pulsieren im Bereich von 1 : 20 bis 2 : 1, bevorzugt bei etwa 1 : 2, liegt.

e) Herstellen der Partikelsuspension indem dem Homogenisat aus Schritt d) das organische Lösungsmittel unter reduziertem Druck bei 20°C bis 50°C, bevorzugt bei 35°C, über einen Zeitraum von 0,5 h bis 5 h, bevorzugt über einen Zeitraum von 2 h bis 3 h entzogen wird.

Alternativ erfolgt nach Schritt e) in einem zusätzlichen Schritt f) das Aufreinigen der Partikelsuspension indem die Partikelsuspension aus Schritt e) ein- bis fünfmal, bevorzugt dreimal abzentrifugiert wird über einen Zeitraum von 20 bis 50 Minuten lang, bevorzugt etwa 30 Minuten lang, bei 0°C bis 30°C, bevorzugt bei etwa 4°C, mit einer Beschleunigung von 10.000 g bis 20.000 g, bevorzugt mit einer Beschleunigung von etwa 14.000 g, wobei der Überstand jeweils durch frisches Aqua purif. ersetzt wird und die Partikel zwischendurch mittels Ultraschall in einem Ultraschallbad resuspendiert werden.

Alternativ erfolgt nach Schritt e) oder nach Schritt f) ein weiterer zusätzlicher Schritt g) zur Filtration der Partikelsuspension durch einen Filter mit einer Porengröße zwischen 2 $\mu$m und 8 $\mu$m, bevorzugt von etwa 5 $\mu$m.

In einem Anwendungsbeispiel im Labormaßstab werden in parallelen Ansätzen in jeweils 5 ml wässrige Phase bei Raumtemperatur jeweils 1 ml organische Phase mit einer Hamiltonspritze unter ständigem Rühren (500rpm) zügig injiziert. Die entstanenen Emulsionen werden gepoolt und auf Eis (max 4°C) oder ungekühlt 3 Minuten (Min.) mit einem Hochleistungs-Dispergiergerät (Ultraturrax®, IKA®-Labortechnik) bei 21.000 Umdrehungen/min weiter emulgiert. Die so erhaltene Emulsion wird mit einem Tip-Sonifier (Sonopuls®, Bandelin) 3 Min. ebenfalls auf Eis (max 4°C) oder ungekühlt bei einer Amplitude von 30-60 % (alternativ 1 Min. bei 25 Watt und 1 min bei 30 Watt) mit oder ohne Pulsieren, d.h. der Einbringung der Ultraschallleistung mit oder ohne Unterbrechungen des Strahlungsflusses, weiter verfeinert. Folgende Pulssequenzen können verwendet werden, beispielsweise: 3 Minuten bei 25-30 Watt bzw. einer Amplitude von 60-75%, und im Intervall jeweils 0,5s Pause und 1 s Schallwirkung.

Der Lösungsmittelanteil (organische Phase) wird anschließend im Rotationsverdampfer unter reduziertem Druck bei 35 °C über ca. 2-3 h abgedampft. Aus der Emulsion entsteht hierbei eine Suspension. Die suspendierten Partikel werden dreimal abzentrifugiert (14.000 g, 30 Min., 4 °C). Der Überstand wird jeweils durch frisches Aqua purif. ersetzt und die Partikel via Ultraschall in einem Ultraschallbad resuspendiert. Final wird die Partikel-Suspension durch einen 5 $\mu$m Nylon-Aufsteckfilter filtriert. Die Suspension kann bis zu 11 Monaten gelagert werden, die reinen Partikel weisen eine Lagerfähigkeit von deutlich über 11 Monaten auf.

Paclitaxel, Docetaxel bzw. Second Generation Taxane wie SB-T-1214 sind kommerziell erhältlich z.B. bei Firma Abmole Biosciense. Poly(lactid-co-glycolid), kurz PLGA, ist kommerziell erhältlich z.B. als Resomer® RG502H, acid terminated, Mw 7,000-17,000) bzw. Resomer® RG504 (lactide:glycolide 50:50, ester terminated, Mw 38,000-54,000) und Resomer® RG504H (acid terminated, lactide:glycolide 50:50, Mw 38,000-54,000) von Boehringer Ingelheim. Poly(vinyl alcohol), kurz PVA, ist kommerziell erhältlich z.B. als Mowiol® 4-88. D-alpha-Tocopheryl Polyethylenglycol 1000 Succinat, kurz TPGS, und DL-$\alpha$-Tocopherol methoxypolyethylene glycol succinate, kurz TPGS-750-M, ist kommerziell erhältlich z.B. von Sigmar Aldrich. Polylactide, kurz PLA ist kommerziell erhältlich z.B. von Polysciences. Poly(lactide -b-ethylene oxide -b-lactide), kurz PLA-PEO-PLA oder LAEOLA, (Verhältnis LA:EO:LA entspricht 2:8:2) und Polylactide, kurz PLA (15 kD) ist kommerziell erhältlich z.B. von Polysciences. HEPES ist kommerziell erhältlich z.B. von Sigmar Aldrich.

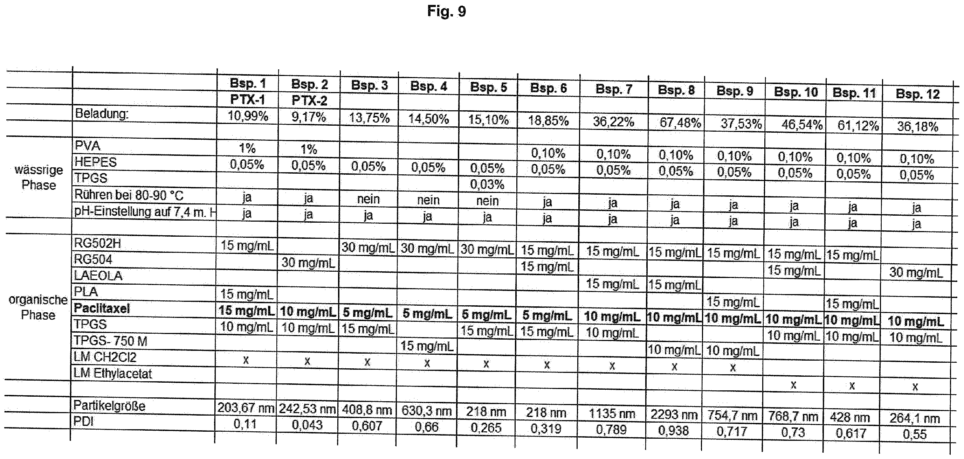

Beispielhaft werden 12 Ausführungsbeispiele zu den Partikeln offenbart, deren Partikelzusammensetzung in der Tabelle Figur 9 übersichtlich dargestellt ist.

**Ausführungsbeispiel 1**

Dieses Beispiel (auch als PTX-1 bezeichnet) zeigt PLGA-PLA-TPGS-PVA-Nanopartikel, in die der Wirkstoff Paclitaxel eingebettet ist. Die Beladung beträgt 10,99 %.

Wässrige Phase: 1% PVA und 0,05% HEPES (2-(4-(2-Hydroxyethyl)-1-piperazinyl)-ethansulfonsäure) werden unter ständigem Rühren bei 80-90°C gelöst, der pH auf 7,4 mit HCl eingestellt und die Lösung nach Abkühlen filtriert. Organische Phase: 15 mg/ml RG502H, 15 mg/ml PLA, 10 mg/ml Paclitaxel und 10 mg/ml TPGS werden in Dichlormethan gelöst und die Lösung filtriert.

Bei diesen Nanopartikeln enthält die Schale das Polymer PVA und der Kern die Polymere RG502H, PLA und TPGS sowie als Wirkstoff Paclitaxel.

Die im Kern befindlichen Polymere liegen in etwa im Verhältnis 3 : 3 : 2 (RG502H : PLA : TPGS) zueinander vor.

Der Gehalt an Paclitaxel, bezogen auf die Partikelgesamtmasse beträgt in etwa 10% (Gewichtsprozent). Die Partikelgröße beträgt 203,67 nm und der PDI 0,11. Die Herstellung erfolgt, wie oben beschrieben.

Figur 1 zeigt das Verneblungsprofil von Ausführungsbeispiel 1.
Figur 2 zeigt die Freisetzungskinetik von Ausführungsbeispiel 1.

**Ausführungsbeispiel 2**

Dieses Beispiel (auch als PTX-2 bezeichnet) zeigt PLGA-PLA-TPGS-PVA Nanopartikel in die der Wirkstoff Paclitaxel eingebettet ist. Die Beladung beträgt 9,17 %. Wässrige Phase: 1% PVA und 0,05% HEPES (2-(4-(2-Hydroxyethyl)-1-piperazinyl)-ethansulfonsäure) werden unter ständigem Rühren bei 80-90°C gelöst, der pH auf 7,4 mit HCl eingestellt und die Lösung nach Abkühlen filtriert. Organische Phase: 30 mg/ml RG504, 10 mg/ml Paclitaxel und 10 mg/ml TPGS werden in Dichlormethan gelöst und die Lösung filtriert.

Bei diesen Nanopartikeln enthält die Schale das Polymer PVA und der Kern die Polymere RG504 und TPGS als Wirkstoff Paclitaxel.

Die im Kern befindlichen Polymere liegen in etwa im Verhältnis 3 : 1 (RG504 : TPGS) zueinander vor.

Der Gehalt an Paclitaxel, bezogen auf die Partikelgesamtmasse beträgt in etwa 10% (Gewichtsprozent). Die Partikelgröße beträgt 242,53 nm und der PDI 0,043. Die Herstellung erfolgt, wie oben beschrieben.

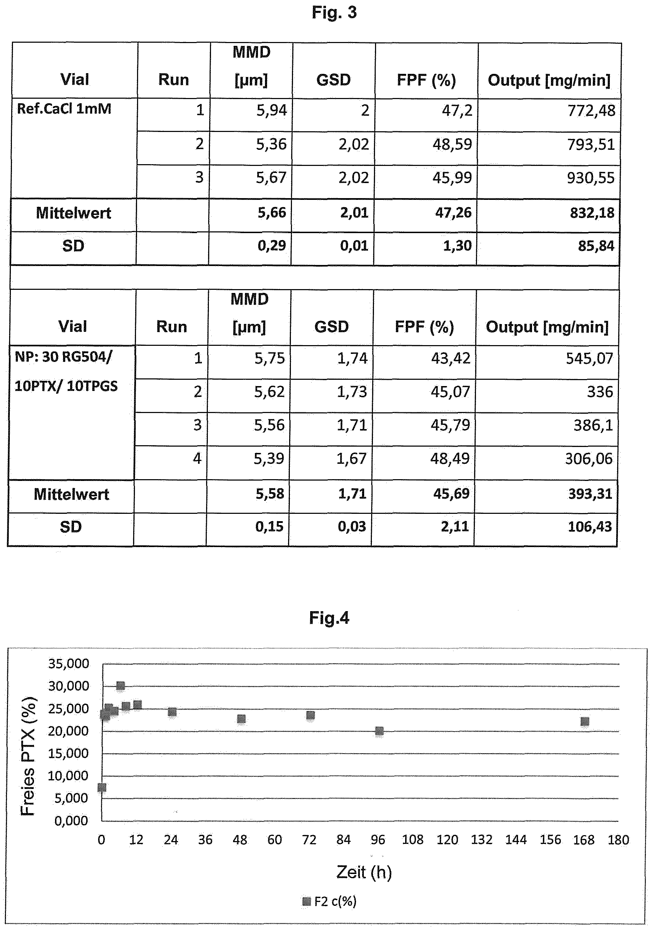

Figur 3 zeigt das Verneblungsprofil von Ausführungsbeispiel 2.
Figur 4 zeigt die Freisetzungskinetik von Ausführungsbeispiel 2.

**Ausführungsbeispiel 3**

Dieses Beispiel zeigt PLGA-TPGS Nanopartikel in die der Wirkstoff Paclitaxel eingebettet ist. Die Beladung beträgt 13,75 %.

Wässrige Phase: 0,05% HEPES (2-(4-(2-Hydroxyethyl)-1-piperazinyl)-ethansulfonsäure) werden unter ständigem Rühren gelöst, der pH auf 7,4 mit HCl eingestellt und die Lösung filtriert.

Organische Phase: 30 mg/ml RG502H, 5 mg/ml Paclitaxel und 15 mg/ml TPGS werden in Dichlormethan gelöst und die Lösung filtriert.

Die Partikelgröße beträgt 408,8 nm und der PDI 0,607. Die Herstellung erfolgt, wie oben beschrieben.

**Ausführungsbeispiel 4**

Dieses Beispiel zeigt PLGA-TPGS Nanopartikel in die der Wirkstoff Paclitaxel eingebettet ist. Die Beladung beträgt 14,5 %.

Wässrige Phase: 0,05% HEPES (2-(4-(2-Hydroxyethyl)-1-piperazinyl)-ethansulfonsäure) werden unter ständigem Rühren gelöst, der pH auf 7,4 mit HCl eingestellt und die Lösung filtriert.

Organische Phase: 30 mg/ml RG502H, 5 mg/ml Paclitaxel und 15 mg/ml TPGS-750 M werden in Dichlormethan gelöst und die Lösung filtriert.

Die Partikelgröße beträgt 630,3 nm und der PDI 0,66. Die Herstellung erfolgt, wie oben beschrieben.

**Ausführungsbeispiel 5**

Dieses Beispiel zeigt PLGA-TPGS-TPGS Nanopartikel in die der Wirkstoff Paclitaxel eingebettet ist. Die Beladung beträgt 15,10 %.

Wässrige Phase: 0,03% TPGS und 0,05% HEPES (2-(4-(2-Hydroxyethyl)-1-piperazinyl)-ethansulfonsäure) werden unter ständigem Rühren gelöst, der pH auf 7,4 mit HCl eingestellt und die Lösung filtriert.

Organische Phase: 30 mg/ml RG502H, 5 mg/ml Paclitaxel und 15 mg/ml TPGS werden in Dichlormethan gelöst und die Lösung filtriert.

Die Partikelgröße beträgt 218 nm und der PDI 0,265. Die Herstellung erfolgt, wie oben beschrieben.

### Ausführungsbeispiel 6

Dieses Beispiel zeigt PLGA-PLGA-TPGS-PVA Nanopartikel in die der Wirkstoff Paclitaxel eingebettet ist. Die Beladung beträgt 18,85 %.

Wässrige Phase: 0,1 % PVA und 0,05% HEPES (2-(4-(2-Hydroxyethyl)-1-piperazinyl)-ethansulfonsäure) werden unter ständigem Rühren bei 80-90°C gelöst, der pH auf 7,4 mit HCl eingestellt und die Lösung nach Abkühlen filtriert. Organische Phase: 15 mg RG504, 15 mg RG502H, 5 mg/ml Paclitaxel und 15 mg/ml TPGS werden in Dichlormethan gelöst und die Lösung filtriert.

Die Partikelgröße beträgt 218 nm und der PDI 0,319. Die Herstellung erfolgt, wie oben beschrieben.

### Ausführungsbeispiel 7

Dieses Beispiel zeigt PLGA-LAEOLA-TPGS-PVA Nanopartikel in die der Wirkstoff Paclitaxel eingebettet ist. Die Beladung beträgt 36,22 %.

Wässrige Phase: 0,1 % PVA und 0,05% HEPES (2-(4-(2-Hydroxyethyl)-1-piperazinyl)-ethansulfonsäure) werden unter ständigem Rühren bei 80-90°C gelöst, der pH auf 7,4 mit HCl eingestellt und die Lösung nach Abkühlen filtriert. Organische Phase: 15 mg/ml RG502H, 15 mg/ml LAEOLA; 10 mg/ml Paclitaxel und 10 mg/ml TPGS werden in Dichlormethan gelöst und die Lösung filtriert.

Die Partikelgröße beträgt 1135 nm und der PDI 0,789. Die Herstellung erfolgt, wie oben beschrieben.

### Ausführungsbeispiel 8

Dieses Beispiel zeigt PLGA-LAEOLA-TPGS-PVA Nanopartikel in die der Wirkstoff Paclitaxel eingebettet ist. Die Beladung beträgt 67,48 %.

Wässrige Phase: 0,1 % PVA und 0,05% HEPES (2-(4-(2-Hydroxyethyl)-1-piperazinyl)-ethansulfonsäure) werden unter ständigem Rühren bei 80-90°C gelöst, der pH auf 7,4 mit HCl eingestellt und die Lösung nach Abkühlen filtriert.

Organische Phase: 15 mg/ml RG502H, 15 mg/ml LAEOLA, 10 mg/ml Paclitaxel und 10 mg/ml TPGS-750-M werden in Dichlormethan gelöst und die Lösung filtriert. Die Partikelgröße beträgt 2293 nm und der PDI 0,938. Die Herstellung erfolgt, wie oben beschrieben.

### Ausführungsbeispiel 9

Dieses Beispiel zeigt PLGA-PLA-TPGS-PVA Nanopartikel in die der Wirkstoff Paclitaxel eingebettet ist. Die Beladung beträgt 37,53 %.

Wässrige Phase: 0,1 % PVA und 0,05% HEPES (2-(4-(2-Hydroxyethyl)-1-piperazinyl)-ethansulfonsäure) werden unter ständigem Rühren bei 80-90°C gelöst, der pH auf 7,4 mit HCl eingestellt und die Lösung nach Abkühlen filtriert.

Organische Phase: 15 mg/ml RG502H, 15 mg/ml PLA, 10 mg/ml Paclitaxel und 10 mg/ml TPGS-750-M werden in Dichlormethan gelöst und die Lösung filtriert.

Die Partikelgröße beträgt 754,7 nm und der PDI 0,717. Die Herstellung erfolgt, wie oben beschrieben.

### Ausführungsbeispiel 10

Dieses Beispiel zeigt PLGA-PLGA-TPGS-PVA Nanopartikel in die der Wirkstoff Paclitaxel eingebettet ist. Die Beladung beträgt 46,54 %.

Wässrige Phase: 0,1 % PVA und 0,05% HEPES (2-(4-(2-Hydroxyethyl)-1-piperazinyl)-ethansulfonsäure) werden unter ständigem Rühren bei 80-90°C gelöst, der pH auf 7,4 mit HCl eingestellt und die Lösung nach Abkühlen filtriert.

Organische Phase: 15 mg/ml RG504, 15 mg/ml RG502H, 10 mg/ml Paclitaxel und 10 mg/ml TPGS werden in Ethylacetat gelöst und die Lösung filtriert.

Die Partikelgröße beträgt 768,7 nm und der PDI 0,73. Die Herstellung erfolgt, wie oben beschrieben.

### Ausführungsbeispiel 11

Dieses Beispiel zeigt PLGA-PLA-TPGS-PVA Nanopartikel in die der Wirkstoff Paclitaxel eingebettet ist. Die Beladung beträgt 61,12 %.

Wässrige Phase: 0,1 % PVA und 0,05% HEPES (2-(4-(2-Hydroxyethyl)-1-piperazinyl)-ethansulfonsäure) werden unter ständigem Rühren bei 80-90°C gelöst, der pH auf 7,4 mit HCl eingestellt und die Lösung nach Abkühlen filtriert.

Organische Phase: 15 mg/ml RG502H, 15 mg/ml PLA, 10 mg/ml Paclitaxel und 10 mg/ml TPGS werden in Ethylacetat gelöst und die Lösung filtriert.

Die Partikelgröße beträgt 428 nm und der PDI 0,617. Die Herstellung erfolgt, wie oben beschrieben.

### Ausführungsbeispiel 12

Dieses Beispiel zeigt PLGA-TPGS-PVA Nanopartikel in die der Wirkstoff Paclitaxel eingebettet ist. Die Beladung beträgt 36,18 %.

Wässrige Phase: 0,1 % PVA und 0,05% HEPES (2-(4-(2-Hydroxyethyl)-1-piperazinyl)-ethansulfonsäure) werden unter ständigem Rühren bei 80-90°C gelöst, der pH auf 7,4 mit HCl eingestellt und die Lösung nach Abkühlen filtriert.

Organische Phase: 30 mg/ml RG504, 10 mg/ml Paclitaxel und 10 mg/ml TPGS werden in Ethylacetat gelöst und die Lösung filtriert.

Die Partikelgröße beträgt 264,1 nm und der PDI 0,55. Die Herstellung erfolgt, wie oben beschrieben.

## Charakterisierung der erfindungsgemäßen Nanopartikel

Nach dem Herstellprozess wird die Partikelsuspension enthaltend den Wirkstoff Paclitaxel oder andere Taxane wie Docetaxel bzw. Second Generation Taxane wie SB-T-1214 wie folgt hinsichtlich Größe und dem PDI (Dispersionsindex, dispersity index oder früher polydispersity index) vermessen und charakterisiert. Dazu werden 10 $\mu$l der Partikelsuspension á ca. 10 mg/ml Nanopartikel nach Aufreinigung entnommen und in 1 ml Aqua. Purif. aufgenommen. In dieser verdünnten Dispersion werden die Partikel dann hinsichtlich Größe und PDI vermessen. Zur Bestimmung der Partikelmasse und Wirkstoffbeladung werden in ausgewogenen Gefäßen z.B. von Eppendorf dreimal 1 ml Partikelsuspension unverdünnt bei 60°C über Nacht eingetrocknet und die Ausbeute gravimetrisch bestimmt. Die Wiederfindung, Beladung und Beladungskapazität erfolgt rückschließend aus der Konzentrationsbestimmung via HPLC. Die Beladungskapazität, d.h. die maximal erreichbare Beladung, bezogen auf die eingesetzte Wirkstoffmenge, liegt durchgängig nie unter 90%.

## Versuchsbeschreibung Vernebelbarkeit/Partikelgröße

Die Partikelsuspension enthaltend den Wirkstoff Paclitaxel bzw. einer der andere Taxane wie Docetaxel bzw. Second Generation Taxane wie SB-T-1214 werden vernebelt. Die Vernebelung erfolgt mit einem Vernebler, wie er im Stand der Technik bekannt ist z.B. dem eFlow® (PARI) unter Zugabe von 1 mM $CaCl_2$ als Elektrolyt. Als Referenz wird 1mM $CaCl_2$-Lösung verwendet.

Der mediane massenbezogene aerodynamische Durchmesser (MMAD) der Aerosoltröpfchen wird mittels Laser Diffraktion (HELOS, Sympatec) ermittelt. Alle Messungen werden im Mie-Modus analysiert. Die geometrische Standardabweichung (GSD) wird durch die gemessenen Werte der Laserdiffraktion wie folgt errechnet:

$$GSD = \sqrt{(d\_(84\%)/d\_(16\%))}$$

d_(n) ist der Tröpfchendurchmesser bei der Prozentzahl n der entsprechenden Größenverteilung.

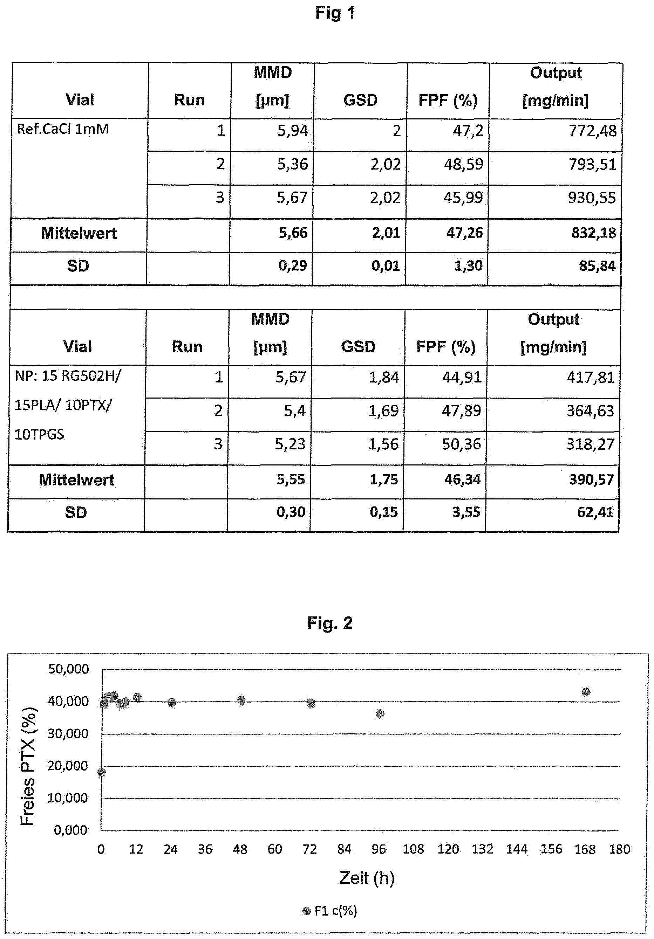

Die "Feine Partikel-Fraktion"(FPF) entspricht der Volumenmenge an Aerosol mit Tröpfchengrößen kleiner als 5,25 $\mu$m. Durch Wiegen des Verneblers vor und nach dem Verneblungsvorgang wird der Output (Aerosol-Massenstrom), sprich die vernebelte Aerosolmasse des Verneblers pro Minute bestimmt (vgl. Fig. 1 und Fig. 3).

## Versuchsbeschreibung Freisetzungskinetik

2 ml Nanopartikelsupension enthaltend den Wirkstoff Paclitaxel bzw. einer der andere Taxane wie Docetaxel bzw. Second Generation Taxane wie SB-T-1214 eingebettet ist, werden unverdünnt abzentrifugiert, der klare Überstand verworfen und der Rückstand mit salinem Phosphat-Puffer (PBS, pH 7,4, 0,1% Sodiumdodecylsulfat) wieder ad 2 ml aufgefüllt.

Die so erhaltenen Proben werden unter ständigem Schütteln bei 37°C inkubiert. An vorbestimmten Zeitpunkten werden jeweils 100$\mu$l entnommen, abzentrifugiert und das Pellet mittels HPLC auf den verbliebenen Wirkstoffgehalt hin untersucht. Aus den so erhaltenen Konzentrationswerten in den Pellets wird die jeweils aktuelle Konzentration im Überstand ("Freies PTX") rechnerisch ermittelt.

Nach dem Herstellprozess werden die polymeren Nanopartikel, in die der Wirkstoff z.B. Paclitaxel oder auch andere Taxane wie Docetaxel bzw. Second Generation Taxane wie SB-T-1214 eingebettet ist, hinsichtlich der Effekte insbesondere ihrer Zytotoxizität, Proliferation und Apoptose auf menschliche IPAH-PASMC-Zellen getestet. Dies sind glatte Muskelzellen aus menschlichen Pulmonalarterien (PASMC), welche aus Lungen von Patienten mit idiopathischer pulmonaler arterieller Hypertension (IPAH) isoliert wurden. Beispielhaft wurden in den in-vitrountersuchungen die beiden Partikelsuspensionen PTX-F1 und PTX-F2 eingesetzt.

## a) Bestimmung der Zytotoxizität

Beispielhaft wurden die beiden Partikelsuspensionen PTX-F1 und PTX-F2 mit unterschiedlicher Konzentration und über verschieden lange Zeiten an menschlichen IPAH-PASMC-Zellen im Hinblick auf ihre Zytotoxizität getestet. Dabei zeigte sich, dass beide nicht toxisch auf die Zellen wirken. Diese zeigten unveränderte Vitalität und keine erkennbaren morphologischen Veränderungen oder ein Absterben.

## b) Bestimmung der Proliferation

Die beispielhaft durchgeführten in-vitro-Bestimmungen der Effekte der Partikelsuspensionen PTX-F1 und PTX-F2 zeigen deutlich die antiproliferative Wirkung an menschlichen IPAH-PASMC-Zellen.

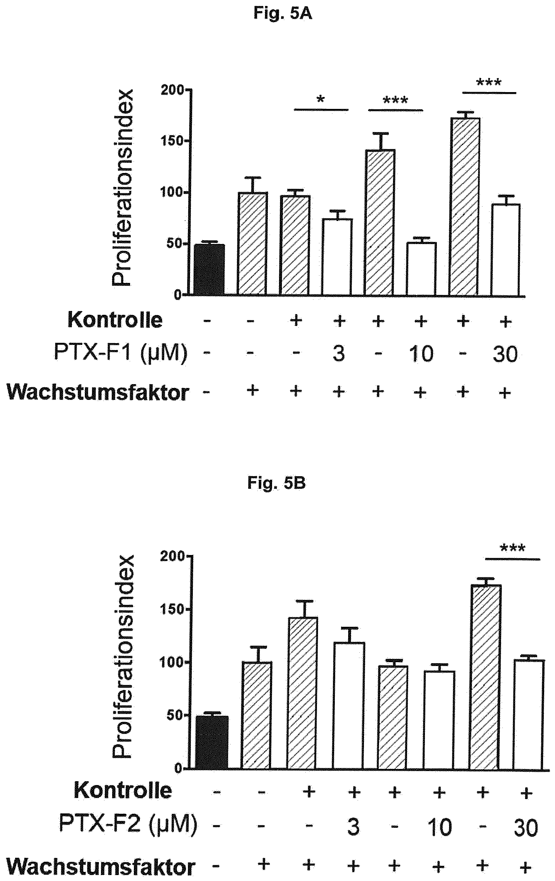

Die Partikelsuspension PTX-F1 verringerte dosisabhängig signifikant die wachstumsfaktor-stimulierte Proliferation von menschlichen IPAH-PASMC-Zellen (Figur 5A). PTX-F2 hingegen zeigte ebenfalls antiproliferative Effekte jedoch bei höheren Konzentrationen (Figur 5B). Als eine Kontrolle wurden Nanopartikel ohne Wirkstoff eingesetzt, die nach oben genanntem Verfahren hergestellt wurden, wobei in Schritt b) kein Wirkstoff zugesetzt wurde.

Nach dem Entzug des Serum über 48 Stunden wurde eine Stimulation der menschlichen IPAH-PASMC-Zellen mit 5%-igem fetalem Kälberserum (FCS) durchgeführt. Anschließend wurden die Zellen 24 Stunden lang mit und ohne Dimethylsulfoxid (DMSO) als Träger mit Partikelsuspension PTX-F1 oder PTX-F2 in Konzentrationen von 3, 10 und 30 μM inkubiert. Anschließend wurde die Proliferation mittels des Bromdesoxyuridin (BrDU)-Proliferationstests gemessen. Zur statistischen Auswertung wurden im Student T-Test die Werte der Testgruppen (PTX-F1 oder PTX-F2) mit den Werten der DMSO-Trägergruppe verglichen. Die Signifikanzen wurden mit * = p<0,05 und *** = p<0,001 festgelegt.

**c) Bestimmung der Apoptose**

Beispielhaft wurden die beiden Partikelsuspensionen PTX-F1 und PTX-F2 an menschlichen IPAH-PASMC-Zellen in Hinblick auf ihre apoptotische Wirkung getestet.

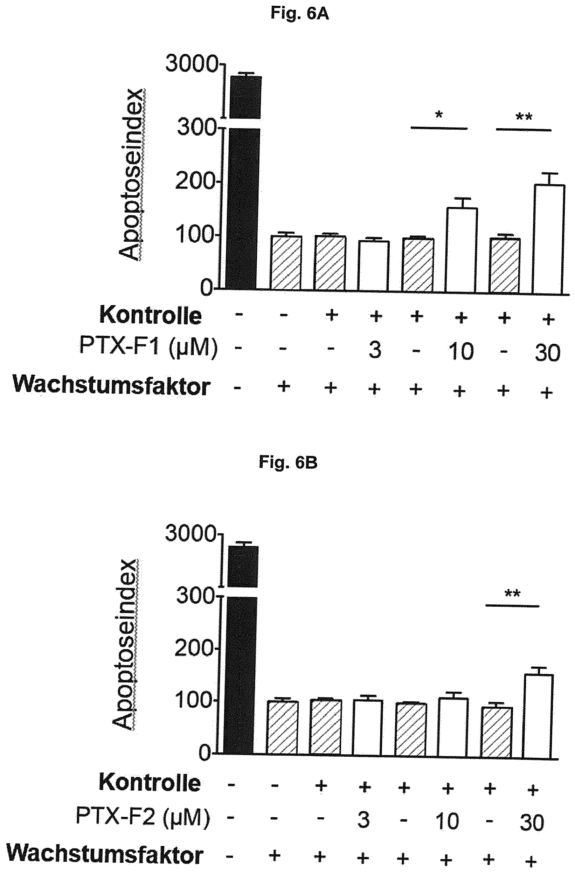

Die Stimulierung mit Wachstumsfaktoren bewirkt eine starke Resistenz der IPAH-PASMC-Zellen gegen Apoptose im Vergleich zu nichtstimulierten IPAH-PASMC-Zellen. Die Partikelsuspension PTX-F1 erhöhte dosisabhängig signifikant die wachstumsfaktor-induzierte Apoptose-Resistenz von menschlichen IPAH-PASMC-Zellen (Figur 6A). PTX-F2 hingegen zeigte eine wachstumsfaktor-induzierte Apoptose-Resistenz nur bei hohen Konzentrationen (Figur 6B). Als eine Kontrolle wurden Nanopartikel ohne Wirkstoff eingesetzt.

Nach dem Entzug des Serum über 48 Stunden wurde eine Stimulation der menschlichen IPAH-PASMC-Zellen mit 5%-igem fetalem Kälberserum (FCS) durchgeführt. Anschließend wurden die Zellen 24 Stunden lang mit und ohne Dimethylsulfoxid (DMSO) als Träger mit PTX-F1 oder PTX-F2 in Konzentrationen von 3, 10 und 30 μM inkubiert. Anschließend wurde die Apoptose mittels des TUNEL-Apoptosetests gemessen. Zur statistischen Auswertung wurden im Student T-Test die Werte der Testgruppen (PTX-F1 oder PTX-F2) mit den Werten der DMSO-Trägergruppe verglichen. Die Signifikanzen wurden mit * = p<0,05 und *** = p<0,001 festgelegt.

**d) Bestimmung der Freisetzungskinetik**

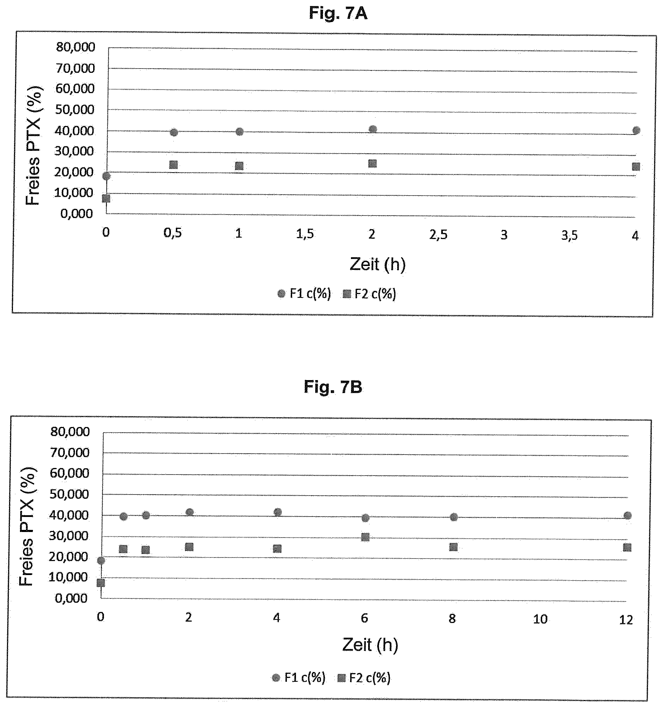

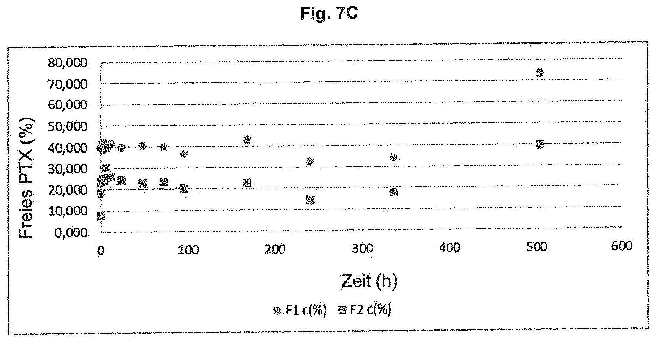

Die beispielhafte Bestimmung der Freisetzungskinetik gemäß der weiter oben offenbarten Versuchsdurchführung anhand von Paclitaxel ergab folgendes Ergebnis (vgl. Fig. 7A, 7B und 7C).

Die erfindungsgemäßen Nanopartikel zeichnen sich dadurch aus, dass sie eine dynamische Freisetzungskinetik für den enthaltenen Wirkstoff aufweisen. Diese dynamische Freisetzungskinetik umfasst mindestens zwei zeitlich aufeinanderfolgende Phasen: Bei der zeitlich ersten Phase handelt es sich um eine sogenannte "Burst-Phase", d.h. einen Zeitraum, in dem eine besonders schnelle Wirkstofffreisetzung erfolgt (Dauer: bis zu etwa 2 Stunden, bevorzugt jedoch kürzer, z.B. etwa 1h), so dass bereits nach kurzer Zeit eine im wesentlichen konstante Konzentration des Wirkstoffs in der Flüssigphase außerhalb der Partikel erreicht wird. Das Erreichen dieser im wesentlichen konstanten Konzentration markiert das für jede individuelle erfindungsgemäße Partikelvariante jeweils individuelle Ende der Burst-Phase. Zeitlich direkt an die Burst-Phase schließt sich sodann die Plateau-Phase an, in der eine langsamere Freisetzung des Wirkstoffs erfolgt, wodurch die in der Burst-Phase erreichte Konzentration in der Flüssigphase weiterhin im wesentlichen aufrechterhalten wird. Diese Plateau-Phase erstreckt sich über einen Zeitraum von bis zu etwa 4 Stunden, bevorzugt über einen Zeitraum von bis zu etwa 12 Stunden, weiter bevorzugt über einen Zeitraum von bis zu etwa 150 Stunden, und ganz besonders bevorzugt über einen Zeitraum von bis zu etwa 400 Stunden (Fig. 7A, 7B und 7C). Infolge dieser Freisetzungskinetik ist es mit den erfindungsgemäßen Nanopartikeln möglich, mindestens einen Wirkstoff z.B. Paclitaxel oder auch andere Taxane wie Docetaxel bzw. Second Generation Taxane wie SB-T-1214 sowohl schnell freizusetzen als auch langanhaltend freizusetzen (Depot-Wirkung), was für eine Behandlung äußerst vorteilhaft ist.

**e) Bestimmung der Lagerstabilität**

Zur Ermittlung der Lagerstabilität wurden die Parameter Größe, Zeta-Potential und Wirkstoffgehalt herangezogen. Dem Fachmann sind die dafür geeigneten Verfahren und Vorgehensweisen aus den Stand der Technik wohlbekannt.

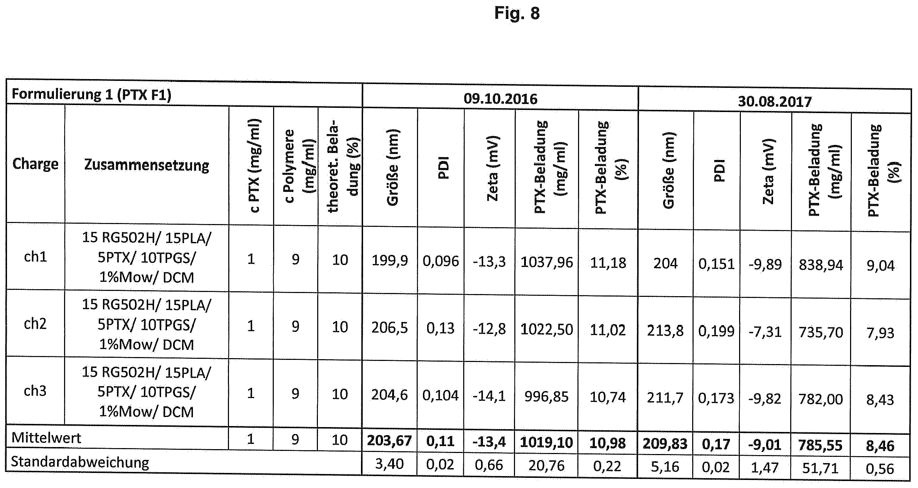

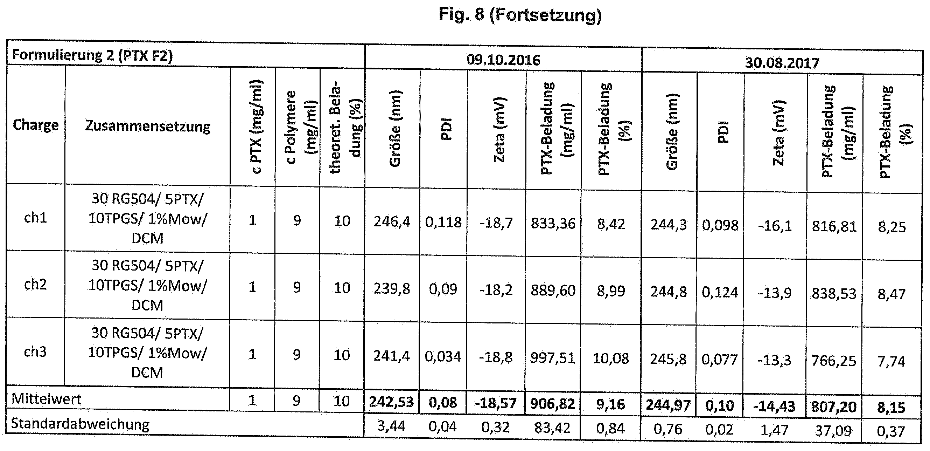

Die Bestimmung der Lagerstabilität erfolgt beispielhaft anhand von Paclitaxelhaltigen erfindungsgemäßen Nanopartikeln. Die Ergebnisse sind in Fig. 8 dargestellt: Nach 11 Monaten Lagerung in Aq. dest. bei 0 - 4°C sind die Partikelgrößen, der PDI und das Zeta-Potential im Wesentlichen unverändert; die prozentuale Beladung mit Wirkstoff (im Beispiel Paclitaxel) ist bei beiden Beispielen etwas gesunken (11% vs. 8,5% bzw. 9,2% vs 8,2%), aber noch außerordentlich gut. Die erfindungsgemäßen Partikel weisen eine sehr gute Lagerungsstabilität auf.

**Abbildungslegenden und Bezugszeichenliste**

Fig. 1    zeigt das Verneblungsprofil der Partikel aus Ausführungsbeispiel 1.
Fig. 2    zeigt die Freisetzungskinetik der Partikel aus Ausführungsbeispiel 1.
Fig. 3    zeigt das Verneblungsprofil der Partikel aus Ausführungsbeispiel 2.
Fig. 4    zeigt die Freisetzungskinetik der Partikel aus Ausführungsbeispiel 2.
Fig. 5A   zeigt Ergebnisse der Messung des Proliferationsindex von PTX-F1.
Fig. 5B   zeigt Ergebnisse der Messung des Proliferationsindex von PTX-F2.
Fig. 6A   zeigt Ergebnisse der Messung des Apoptose-Index von PTX-F1.

Fig. 6B zeigt Ergebnisse der Messung des Apoptose-Index von PTX-F2.

Fig. 7A zeigt die Freisetzungskinetik von PTX-F1/PTX-F2 von bis zu 4 Stunden.

Fig. 7B zeigt die Freisetzungskinetik von PTX-F1/PTX-F2 von bis zu 12 Stunden.

Fig. 7C zeigt die Freisetzungskinetik von PTX-F1/PTX-F2 von bis zu 550 Stunden.

Fig. 8 zeigt die Messergebnisse der beispielhaft vorgenommenen Stabilitätsuntersuchungen anhand der Zubereitungen PTX-F1 und PTX-F2.

Fig. 9 zeigt eine Zusammenstellung der hergestellten Partikel gemäß Ausführungsbeispiele 1-12 inklusive der Zubereitungen PTX-F1 und PTX-F2.

**Patentansprüche**

1. Nanopartikel umfassend mindestens drei Polymere, unabhängig voneinander ausgewählt aus der Liste umfassend PLGA, PLA, TPGS, TPGS-750M, PVA, LAEOLA/PLA-PEO-PLA sowie mindestens einen Wirkstoff, ausgewählt aus der Liste umfassend Paclitaxel, Docetaxel sowie SB-T-1214, **dadurch gekennzeichnet dass**

   i) die Partikel einen Kern-Schale-Aufbau aufweisen, wobei der Kern den mindestens einen Wirkstoff und mindestens zwei Polymere enthält, wobei die Polymere unabhängig voneinander ausgewählt sind aus der Liste umfassend RG502H, RG504, PLA, LAEOLA/PLA-PEO-PLA, TPGS sowie TPGS-750M, und
   ii) die Schale PVA und/oder TPGS enthält, und
   iii) die Nanopartikel vernebelbar sind.

2. Nanopartikel nach Anspruch 1, **dadurch gekennzeichnet, dass** die Partikel eine dynamische Freisetzungskinetik für den mindestens einen medizinischen Wirkstoff aufweisen, wobei diese dynamische Freisetzungskinetik mindestens zwei zeitlich aufeinanderfolgende Phasen umfasst, wobei in einer ersten anfänglichen Burst-Phase von bis zu etwa 2 Stunden, bevorzugt bis zu etwa 1 Stunde, eine schnelle Freisetzung erfolgt, wodurch eine im wesentlichen konstante Konzentration in der Flüssigphase außerhalb der Partikel bis zum Ende der Burst-Phase erreicht wird, und in einer an die Burst-Phase zeitlich anschließenden Plateau-Phase, die sich über einen Zeitraum von bis zu etwa 4 Stunden, bevorzugt über einen Zeitraum von bis zu etwa 12 Stunden, weiter bevorzugt über einen Zeitraum von bis zu etwa 150 Stunden, und ganz besonders bevorzugt über einen Zeitraum von bis zu etwa 400 Stunden erstreckt, eine langsame Freisetzung erfolgt, wodurch die in der Burst-Phase erreichte Konzentration in der Flüssigphase im Wesentlichen aufrechterhalten wird.

3. Nanopartikel nach Anspruch 1 oder Anspruch 2, **dadurch gekennzeichnet, dass** die Beladung mit dem mindestens einen medizinischen Wirkstoff, bezogen auf das Partikelgesamtgewicht, zwischen 1% und 80%, bevorzugt zwischen 3% und 70%, besonders bevorzugt zwischen 5% und 50%, ganz besonders bevorzugt zwischen 10% und 40% beträgt.

4. Nanopartikel nach einem der vorstehenden Ansprüche 1 bis 3, **dadurch gekennzeichnet, dass** die Schale das Polymer PVA enthält und der Kern die Polymere RG502H, PLA und TPGS sowie als medizinischen Wirkstoff Paclitaxel enthält, wobei die im Kern befindlichen Polymere in etwa im Verhältnis 3 : 3 : 2 (RG502H : PLA : TPGS) zueinander vorliegen, und der Gehalt an Paclitaxel, bezogen auf die Partikelgesamtmasse in etwa 10% (Gewichtsprozent) beträgt.

5. Nanopartikel nach einem der vorstehenden Ansprüche 1 bis 3, **dadurch gekennzeichnet, dass** die Schale das Polymer PVA enthält und der Kern die Polymere RG504 und TPGS sowie als medizinischen Wirkstoff Paclitaxel enthält, wobei die im Kern befindlichen Polymere in etwa im Verhältnis 3 : 1 (RG504 : TPGS) zueinander vorliegen, und der Gehalt an Paclitaxel, bezogen auf die Partikelgesamtmasse in etwa 10% (Gewichtsprozent) beträgt.

6. Nanopartikel nach einem der vorstehenden Ansprüche 1 bis 5, **dadurch gekennzeichnet, dass** sie eine Lagerungsstabilität von bis zu 11 Monaten in Dispersion und/oder über 11 Monaten in Substanz/Reinform aufweisen, wobei sich die Lagerungsstabilität der Partikel in einer im wesentlichen konstanten Größe der Nanopartikel und einem im wesentlichen konstanten Zetapotential der Nanopartikel und einer Abnahme der Wirkstoffkonzentration von maximal 30%, bevorzugt von maximal 25%, weiter bevorzugt von maximal 15% und ganz besonders bevorzugt von maximal etwa 10% darstellt.

7. Verfahren zur Herstellung der Nanopartikel nach einem der vorstehenden Ansprüche 1 bis 3 umfassend die Schritte:

   a) Herstellen einer wässrigen Phase umfassend 0,01 % bis 1%, bevorzugt 0,05%, eines Polymers, ausgewählt aus der Liste umfassend PVA sowie TPGS und/oder der Puffersubstanz HEPES, wobei das Polymer und/oder die Puffersubstanz HEPES unter Rühren gelöst wird und der pH-Wert auf einen Wert zwischen 7 und 8, bevorzugt auf etwa 7,4 eingestellt und die wässrige Phase auf 19°C bis 25°C, bevorzugt auf 22°C abgekühlt wird.

b) Herstellen einer organischen Phase umfassend

- mindestens zwei Polymeren ausgewählt aus der Liste umfassend RG502H, RG504, LAEOLA, PLA, TPGS sowie TPGS-750M wobei die Konzentration der mindestens zwei Polymere 10 mg/ml bis 30 mg/ml, bevorzugt etwa 15 mg/ml beträgt
- mindestens einen Wirkstoff ausgewählt aus der Liste umfassend Paclitaxel, Docetaxel sowie SB-T-1214 wobei die Konzentration des mindestens einen Wirkstoffs 5 mg/ml bis 15 mg/ml, bevorzugt etwa 10 mg/ml beträgt

wobei die mindestens zwei Polymere und der mindestens eine Wirkstoff in einem unpolaren aprotischen organischen Lösungsmittel, ausgewählt aus der Liste umfassend Methylenchlorid sowie Ethylacetat gelöst werden.

c) Mischen von 5 Teilen der wässrigen Phase aus Schritt a) mit einem Teil der organischen Phase aus Schritt b) sodass eine Emulsion entsteht, die bei 0°C bis 30°C, bevorzugt bei 0°C bis 4°C für einen Zeitraum von 1 bis 5 Minuten, bevorzugt etwa 3 Minuten mit einem Hochleistungs-Dispergiergerät bei 10.000 Umdrehungen/Min. bis 30.000 Umdrehungen/Min., bevorzugt bei etwa 21.000 Umdrehungen/Min weiter emulgiert wird, so dass die Teilchengröße verringert wird.

d) Homogenisieren der Emulsion aus Schritt c) mit einem Ultraschall-Homogenisator bei 0°C bis 30°C, bevorzugt bei 0°C bis 4°C,

e) Herstellen der Partikelsuspension indem dem Homogenisat aus Schritt d) das organische Lösungsmittel unter reduziertem Druck bei 20°C bis 50°C, bevorzugt bei 35 °C, über einen Zeitraum von 0,5 h bis 5 h, bevorzugt über einen Zeitraum von 2 h bis 3 h entzogen wird.

8. Verfahren zur Herstellung der Nanopartikel gemäß Anspruch 7 **dadurch gekennzeichnet dass** in Schritt c) das Mischen der wässrige Phase nach Schritt a) mit der organischen Phase nach Schritt b) bei einer Temperatur von 19-22°C und unter ständige Rühren bei einer Rührgeschwindigkeit zwischen 300 Umdrehungen/Min. und 700 Umdrehungen/Min., bevorzugt etwa 500 Umdrehungen/Min.) erfolgt.

9. Verfahren zur Herstellung der Nanopartikel gemäß Anspruch 7 **dadurch gekennzeichnet dass** in Schritt d) das Homogenisieren der Emulsion aus Schritt c) über einen Zeitraum von 0,5 bis 2 Minuten, bevorzugt 1 Minute, mit einer Leistung von etwa 25 Watt und anschließend 0,5 bis 2 Minuten, bevorzugt 1 Minute, mit einer Leistung von etwa 30 Watt erfolgt.

10. Verfahren zur Herstellung der Nanopartikel gemäß Anspruch 7 **dadurch gekennzeichnet dass** in Schritt d) das Homogenisieren der Emulsion aus Schritt c) über einen Zeitraum von 2 bis 5 Minuten, bevorzugt 3 Minuten, mit einer Leistung von 20 bis 40 Watt, bevorzugt mit einer Leistung von 25 bis 30 Watt oder mit einer Amplitude von 20% bis 90%, bevorzugt mit einer Amplitude von 30% bis 75%, besonders bevorzugt mit einer Amplitude von 60% bis 75% erfolgt.

11. Verfahren zur Herstellung der Nanopartikel gemäß Anspruch 7 **dadurch gekennzeichnet dass** in Schritt d) das Homogenisieren der Emulsion aus Schritt c) mit oder ohne Pulsieren erfolgt, wobei beim Pulsieren das Verhältnis "Dauer der Pause" und "Dauer der Schallwirkung" im Bereich von 1:20 bis 2:1, bevorzugt bei etwa 1:2, liegt.

12. Verfahren zur Herstellung der Nanopartikel gemäß der Ansprüche 7 bis 11 **dadurch gekennzeichnet dass** nach Schritt e) ein weiterer Schritt f) durchgeführt wird zur Aufreinigung der Partikelsuspension indem die Partikelsuspension aus Schritt e) ein- bis fünfmal, bevorzugt dreimal abzentrifugiert wird über einen Zeitraum von 20 bis 50 Minuten, bevorzugt etwa 30 Minuten, bei einer Temperatur von 0°C bis 30 °C, bevorzugt bei etwa 4 °C, mit einer Beschleunigung von 10.000 g bis 20.000 g, bevorzugt mit einer Beschleunigung von etwa 14.000 g, wobei der Überstand jeweils durch frisches Aqua purif. ersetzt wird und die Partikel zwischendurch mittels Ultraschall in einem Ultraschallbad resuspendiert werden.

13. Verfahren zur Herstellung der Nanopartikel gemäß der Ansprüche 7 bis 12 **dadurch gekennzeichnet dass** nach Schritt e) oder nach Schritt f) ein weiterer Schritt g) durchgeführt wird zur Filtration durch einen Filter mit einer Porengröße zwischen 2 $\mu$m und 8 $\mu$m, bevorzugt von etwa 5 $\mu$m.

14. Verwendung der Nanopartikel nach einem der Ansprüche 1 bis 6 zur inhalativen Applikation von mindestens einem Wirkstoff ausgewählt aus der Liste umfassend Paclitaxel, Docetaxel sowie SB-T-1214 in einem Vernebler.

15. Verwendung der Nanopartikel nach einem der Ansprüche 1 bis 6 zur Behandlung der pulmonalen Hypertonie.

## Fig 1

| Vial | Run | MMD [µm] | GSD | FPF (%) | Output [mg/min] |
|---|---|---|---|---|---|
| Ref.CaCl 1mM | 1 | 5,94 | 2 | 47,2 | 772,48 |
| | 2 | 5,36 | 2,02 | 48,59 | 793,51 |
| | 3 | 5,67 | 2,02 | 45,99 | 930,55 |
| Mittelwert | | 5,66 | 2,01 | 47,26 | 832,18 |
| SD | | 0,29 | 0,01 | 1,30 | 85,84 |

| Vial | Run | MMD [µm] | GSD | FPF (%) | Output [mg/min] |
|---|---|---|---|---|---|
| NP: 15 RG502H/ 15PLA/ 10PTX/ 10TPGS | 1 | 5,67 | 1,84 | 44,91 | 417,81 |
| | 2 | 5,4 | 1,69 | 47,89 | 364,63 |
| | 3 | 5,23 | 1,56 | 50,36 | 318,27 |
| Mittelwert | | 5,55 | 1,75 | 46,34 | 390,57 |
| SD | | 0,30 | 0,15 | 3,55 | 62,41 |

## Fig. 2

## Fig. 3

| Vial | Run | MMD [μm] | GSD | FPF (%) | Output [mg/min] |
|---|---|---|---|---|---|
| Ref.CaCl 1mM | 1 | 5,94 | 2 | 47,2 | 772,48 |
| | 2 | 5,36 | 2,02 | 48,59 | 793,51 |
| | 3 | 5,67 | 2,02 | 45,99 | 930,55 |
| Mittelwert | | 5,66 | 2,01 | 47,26 | 832,18 |
| SD | | 0,29 | 0,01 | 1,30 | 85,84 |

| Vial | Run | MMD [μm] | GSD | FPF (%) | Output [mg/min] |
|---|---|---|---|---|---|
| NP: 30 RG504/ 10PTX/ 10TPGS | 1 | 5,75 | 1,74 | 43,42 | 545,07 |
| | 2 | 5,62 | 1,73 | 45,07 | 336 |
| | 3 | 5,56 | 1,71 | 45,79 | 386,1 |
| | 4 | 5,39 | 1,67 | 48,49 | 306,06 |
| Mittelwert | | 5,58 | 1,71 | 45,69 | 393,31 |
| SD | | 0,15 | 0,03 | 2,11 | 106,43 |

## Fig.4

## Fig. 5A

## Fig. 5B

Fig. 6A

Fig. 6B

## Fig. 7A

## Fig. 7B

## Fig. 7C

**Fig. 8**

| Formulierung 1 (PTX F1) | | c PTX (mg/ml) | c Polymere (mg/ml) | theoret. Bela-dung (%) | 09.10.2016 | | | | | 30.08.2017 | | | | |
|---|---|---|---|---|---|---|---|---|---|---|---|---|---|---|
| Charge | Zusammensetzung | c PTX (mg/ml) | c Polymere (mg/ml) | theoret. Bela-dung (%) | Größe (nm) | PDI | Zeta (mV) | PTX-Beladung (mg/ml) | PTX-Beladung (%) | Größe (nm) | PDI | Zeta (mV) | PTX-Beladung (mg/ml) | PTX-Beladung (%) |
| ch1 | 15 RG502H/ 15PLA/ 5PTX/ 10TPGS/ 1%Mow/ DCM | 1 | 9 | 10 | 199,9 | 0,096 | -13,3 | 1037,96 | 11,18 | 204 | 0,151 | -9,89 | 838,94 | 9,04 |
| ch2 | 15 RG502H/ 15PLA/ 5PTX/ 10TPGS/ 1%Mow/ DCM | 1 | 9 | 10 | 206,5 | 0,13 | -12,8 | 1022,50 | 11,02 | 213,8 | 0,199 | -7,31 | 735,70 | 7,93 |
| ch3 | 15 RG502H/ 15PLA/ 5PTX/ 10TPGS/ 1%Mow/ DCM | 1 | 9 | 10 | 204,6 | 0,104 | -14,1 | 996,85 | 10,74 | 211,7 | 0,173 | -9,82 | 782,00 | 8,43 |
| Mittelwert | | 1 | 9 | 10 | **203,67** | **0,11** | **-13,4** | **1019,10** | **10,98** | **209,83** | **0,17** | **-9,01** | **785,55** | **8,46** |
| Standardabweichung | | | | | 3,40 | 0,02 | 0,66 | 20,76 | 0,22 | 5,16 | 0,02 | 1,47 | 51,71 | 0,56 |

## Fig. 8 (Fortsetzung)

| Formulierung 2 (PTX F2) | | | | | 09.10.2016 | | | | | 30.08.2017 | | | | |
|---|---|---|---|---|---|---|---|---|---|---|---|---|---|---|
| Charge | Zusammensetzung | c PTX (mg/ml) | c Polymere (mg/ml) | theoret. Beladung (%) | Größe (nm) | PDI | Zeta (mV) | PTX-Beladung (mg/ml) | PTX-Beladung (%) | Größe (nm) | PDI | Zeta (mV) | PTX-Beladung (mg/ml) | PTX-Beladung (%) |
| ch1 | 30 RG504/ 5PTX/ 10TPGS/ 1%Mow/ DCM | 1 | 9 | 10 | 246,4 | 0,118 | -18,7 | 833,36 | 8,42 | 244,3 | 0,098 | -16,1 | 816,81 | 8,25 |
| ch2 | 30 RG504/ 5PTX/ 10TPGS/ 1%Mow/ DCM | 1 | 9 | 10 | 239,8 | 0,09 | -18,2 | 889,60 | 8,99 | 244,8 | 0,124 | -13,9 | 838,53 | 8,47 |
| ch3 | 30 RG504/ 5PTX/ 10TPGS/ 1%Mow/ DCM | 1 | 9 | 10 | 241,4 | 0,034 | -18,8 | 997,51 | 10,08 | 245,8 | 0,077 | -13,3 | 766,25 | 7,74 |
| Mittelwert | | 1 | 9 | 10 | 242,53 | 0,08 | -18,57 | 906,82 | 9,16 | 244,97 | 0,10 | -14,43 | 807,20 | 8,15 |
| Standardabweichung | | | | | 3,44 | 0,04 | 0,32 | 83,42 | 0,84 | 0,76 | 0,02 | 1,47 | 37,09 | 0,37 |

EP 3 453 385 A1

**Fig. 9**

| | | Bsp. 1 PTX-1 | Bsp. 2 PTX-2 | Bsp. 3 | Bsp. 4 | Bsp. 5 | Bsp. 6 | Bsp. 7 | Bsp. 8 | Bsp. 9 | Bsp. 10 | Bsp. 11 | Bsp. 12 |
|---|---|---|---|---|---|---|---|---|---|---|---|---|---|
| | Beladung: | 10,99% | 9,17% | 13,75% | 14,50% | 15,10% | 18,85% | 36,22% | 67,48% | 37,53% | 46,54% | 61,12% | 36,18% |
| wässrige Phase | PVA | 1% | 1% | | | | 0,10% | 0,10% | 0,10% | 0,10% | 0,10% | 0,10% | 0,10% |
| | HEPES | 0,05% | 0,05% | 0,05% | 0,05% | 0,05% | 0,05% | 0,05% | 0,05% | 0,05% | 0,05% | 0,05% | 0,05% |
| | TPGS | | | | | 0,03% | | | | | | | |
| | Rühren bei 80-90 °C | ja | ja | nein | nein | nein | ja | ja | ja | ja | ja | ja | ja |
| | pH-Einstellung auf 7,4 m. H | ja | ja | ja | ja | ja | ja | ja | ja | ja | ja | ja | ja |
| organische Phase | RG502H | 15 mg/mL | | 30 mg/mL | 30 mg/mL | 30 mg/mL | 15 mg/mL | 15 mg/mL | 15 mg/mL | 15 mg/mL | 15 mg/mL | 15 mg/mL | |
| | RG504 | | 30 mg/mL | | | | | 15 mg/mL | | | | | |
| | LAEOLA | | | | | | | | | | 15 mg/mL | | 30 mg/mL |
| | PLA | 15 mg/mL | | | | | | | 15 mg/mL | 15 mg/mL | | | |
| | **Paclitaxel** | **15 mg/mL** | **10 mg/mL** | **5 mg/mL** | **5 mg/mL** | **5 mg/mL** | **5 mg/mL** | **10 mg/mL** | **10 mg/mL** | **10 mg/mL** | **10 mg/mL** | **10 mg/mL** | **10 mg/mL** |
| | TPGS | 10 mg/mL | 10 mg/mL | 15 mg/mL | | 15 mg/mL | 15 mg/mL | 10 mg/mL | | | 10 mg/mL | 10 mg/mL | 10 mg/mL |
| | TPGS- 750 M | | | | 15 mg/mL | | | | 10 mg/mL | 10 mg/mL | | | |
| | LM CH2Cl2 | x | x | x | x | x | x | x | x | x | | | |
| | LM Ethylacetat | | | | | | | | | | x | x | x |
| | Partikelgröße | 203,67 nm | 242,53 nm | 408,8 nm | 630,3 nm | 218 nm | 218 nm | 1135 nm | 2293 nm | 754,7 nm | 768,7 nm | 428 nm | 264,1 nm |
| | PDI | 0,11 | 0,043 | 0,607 | 0,66 | 0,265 | 0,319 | 0,789 | 0,938 | 0,717 | 0,73 | 0,617 | 0,55 |

**EP 3 453 385 A1**

Europäisches Patentamt
European Patent Office
Office européen des brevets

# EUROPÄISCHER RECHERCHENBERICHT

Nummer der Anmeldung

EP 17 18 9860

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | MU L ET AL: "Vitamin E TPGS used as emulsifier in the solvent evaporation/extraction technique for fabrication of polymeric nanospheres for controlled release of paclitaxel (Taxol)", JOURNAL OF CONTROLLED RELEASE, ELSEVIER, AMSTERDAM, NL, Bd. 80, Nr. 1, 4. Januar 2016 (2016-01-04), Seiten 129-144, XP029377498, ISSN: 0168-3659, DOI: 10.1016/S0168-3659(02)00025-1 * das ganze Dokument * * Zusammenfassung * ----- | 1-15 | INV. A61K9/50 A61K9/51 |
| X | FENG S-S ET AL: "Chemotherapeutic engineering: Application and further development of chemical engineering principles for chemotherapy of cancer and other diseases", CHEMICAL ENGINEERING SCI, OXFORD, GB, Bd. 58, Nr. 18, 1. September 2003 (2003-09-01), Seiten 4087-4114, XP004453411, ISSN: 0009-2509, DOI: 10.1016/S0009-2509(03)00234-3 * das ganze Dokument * * Zusammenfassung * ----- | 1-15 | RECHERCHIERTE SACHGEBIETE (IPC)  A61K |
| X | WO 2010/005721 A2 (BIND BIOSCIENCES INC [US]; ZALE STEPHEN E [US]; TROIANO GREG [US]; ALI) 14. Januar 2010 (2010-01-14) * das ganze Dokument * * Ansprüche 1-46; Beispiele 1-19 * ----- | 1-15 | |

Der vorliegende Recherchenbericht wurde für alle Patentansprüche erstellt

| Recherchenort | Abschlußdatum der Recherche | Prüfer |
|---|---|---|
| München | 23. November 2017 | Felder, Christian |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
...............................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 17 18 9860

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

23-11-2017

| Im Recherchenbericht angeführtes Patentdokument | Datum der Veröffentlichung | Mitglied(er) der Patentfamilie | Datum der Veröffentlichung |
|---|---|---|---|
| WO 2010005721 A2 | 14-01-2010 | AU 2009268923 A1 | 14-01-2010 |
| | | CA 2728176 A1 | 14-01-2010 |
| | | CN 104997732 A | 28-10-2015 |
| | | EA 201170038 A1 | 30-08-2011 |
| | | EA 201170039 A1 | 30-12-2011 |
| | | EP 2309989 A2 | 20-04-2011 |
| | | EP 2309990 A2 | 20-04-2011 |
| | | EP 2774608 A1 | 10-09-2014 |
| | | ES 2462090 T3 | 22-05-2014 |
| | | HK 1211492 A1 | 27-05-2016 |
| | | JP 6104289 B2 | 29-03-2017 |
| | | JP 2012501964 A | 26-01-2012 |
| | | JP 2012501965 A | 26-01-2012 |
| | | JP 2015134759 A | 27-07-2015 |
| | | JP 2016041756 A | 31-03-2016 |
| | | KR 20110036810 A | 11-04-2011 |
| | | KR 20160116062 A | 06-10-2016 |
| | | US 2010068285 A1 | 18-03-2010 |
| | | US 2010068286 A1 | 18-03-2010 |
| | | US 2011274759 A1 | 10-11-2011 |
| | | US 2012276162 A1 | 01-11-2012 |
| | | US 2012288541 A1 | 15-11-2012 |
| | | US 2013230567 A1 | 05-09-2013 |
| | | US 2013236500 A1 | 12-09-2013 |
| | | US 2013243827 A1 | 19-09-2013 |
| | | US 2013251757 A1 | 26-09-2013 |
| | | US 2013280339 A1 | 24-10-2013 |
| | | US 2013302432 A1 | 14-11-2013 |
| | | US 2013302433 A1 | 14-11-2013 |
| | | US 2014356444 A1 | 04-12-2014 |
| | | US 2016338959 A1 | 24-11-2016 |
| | | WO 2010005721 A2 | 14-01-2010 |
| | | WO 2010005723 A2 | 14-01-2010 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

**In der Beschreibung aufgeführte Patentdokumente**

*   US 20140271871 A1

**In der Beschreibung aufgeführte Nicht-Patentliteratur**

*   **BERNABEU et al.** Paclitaxel: What has been done and the challenges remain ahead. *Int J Pharm.,* 2017, vol. 526 (1-2), 474-495